# EUROPEAN PATENT APPLICATION

(11) **EP 4 847 625 A2**
(43) Date of publication of application: **02.12.2026**
(21) Application number: 26210486.2
(22) Date of filing: 02.02.2024
(51) Int. Cl.: C12P 21/02

(54) **METHOD OF CHEMICAL SYNTHESIS OF SINGLE-CHAIN ANTIBODY FRAGMENTS AND PRODUCTS THEREBY OBTAINED**

(30) Priority: 09.02.2023 EP 23425005
(62) Document number(s) of the earlier application(s) in accordance with Art. 76 EPC: 24703933.2 / 4 662 235
(71) Applicant: Figini, Mariangela, 20129 Milano (IT); GLYTECH, INC., Kyoto 600-8813 (JP)
(72) Inventor: FIGINI, Mariangela, 20129 Milano (IT); LUISON, Elena, 20045 Lainate (IT)
(74) Representative: M. Zardi & Co S.A.

(57) **Abstract**

A new method is disclosed for the protein full-chemical synthesis of single-chain antibody fragments (scFv); the obtained products are structurally close and functionally equivalent to their biological counterpart, being however, advantageously free of impurities and more reproducible in properties. The method includes the general steps of: (a) separately synthetizing sub-sequences (peptide fragments) (b) sequentially assembling the sub-sequences obtained in step (a) in the order as they appear in the target scFv amino acid sequence, and (c) performing oxidative folding of the assembled whole peptide molecule obtained in step (b) and dialyzing the resulting product in a buffer.

## Description

### FIELD OF THE INVENTION

The invention relates to the field of preparation of antibodies for use in human therapy and diagnostic; in summary, the presented invention demonstrates the use of protein chemical synthesis as a general platform for the production of highly functional antibody fragments.

### STATE OF THE ART

Biologics are drugs made from complex molecules manufactured using living microorganisms, plants, or animal cells. Many are produced using recombinant DNA technology. They are sometimes referred to as biopharmaceuticals or biological drugs. Biopharmaceutical drugs represent an increasing sector in the pharmaceutical field: for example, within the last recent years, they have accounted for 30% of all new pharmaceutical sales in the United States, for therapeutic and diagnostic use. The rising demand for new biopharmaceuticals requires increased production capacities as well as new production processes that exhibit increased space-time yields and shortened development times which also implies the use of suitable expression systems. Biological drugs include interleukins, vaccines and the best-known examples of these are antibodies, which bind to the surface of cells in the body and are used extensively in the treatment of cancer.

Antibodies are the key detection elements in research, diagnostics, and therapeutics. Until now, they are the fastest growing class of therapeutic protein agents. Besides manufacturing of the classical IgG antibody format, a rapidly growing number of recombinant antibodies are currently being made available. Antibody fragments are also of increasing clinical importance and several methods have evolved to meet these demands.

Small recombinant Ab (rAb) fragments are increasingly being used as alternatives to full monoclonal antibodies (mAbs) in some medical diagnostic and therapeutic applications. A variety of rAb formats have been tailored for specific applications including engineered modifications to antigen binding, valency, and molecular weight (MW). One of the most popular types of rAbs are single-chain variable fragments (scFvs) as they have been successfully modified into a number of different Ab formats and are easily expressed by several expression systems. scFvs are complex peptide products, containing a high number of aminoacids, generally ranging from 230 to 280, and structured in a tridimensional structure. They contain the complete antigen binding site, which includes the variable heavy (VH) and variable light (VL) domains, of an antibody. The VH domain is linked to a VL domain by an introduced flexible polypeptide linker.

rAb fragments have been expressed in a wide variety of hosts including prokaryotes, such as E. coli and B. subtilis, and eukaryotes, including S. cerevisiae, Pichia pastoris, insect cells, plants and mammalian cells. For therapeutic purposes, large doses of Ab are required, and in some cases exceed a gram per patient per year. Thus, there is a need to develop production systems to make these molecules efficiently and cost effectively.

All the above systems are able to produce antibodies and antibodies fragments using living microorganisms; the resulting products are "biological drugs", which are subjected to the requirements relating to the production of products derived by rDNA methodology and to general recommendations for the quality control of biological products (e.g. GMP Directive 91/356/EEC and Directive 90/219/EEC on the contained use of genetically modified micro-organisms).

Despite their recognized and unique utility, biological products suffer a number of limitations associated with their natural origin: most of them are complex mixtures that are not easily identified or characterized; they tend to be heat sensitive and susceptible to microbial contamination, which requires the use of use aseptic principles from initial manufacturing steps. Furthermore, compared to drugs obtained by chemical synthesis, they are more difficult, and sometimes impossible, to fully characterize by testing methods available in the laboratory, and some of the components of a finished biologic may remain unknown. The quality of these products is strongly dependent on the manufacturing process; therefore, manufacturers must ensure product consistency, quality, and purity by ensuring that the manufacturing process remains substantially the same over time. By contrast, a drug manufacturer can change the manufacturing process extensively and analyze the finished product to establish that it is the same as before the manufacturing change.

In addition, the living systems used to produce biologics can be sensitive to very minor changes in the manufacturing process. Small process differences can significantly affect the nature of the finished biologic and, most importantly, the way it functions in the body. To ensure that a manufacturing process remains the same over time, biologics manufacturers must tightly control the source and nature of starting materials, and consistently employ hundreds of process controls that assure predictable manufacturing outcomes. Finally, process controls for biologics are established separately for each unique manufacturing process/product and are not applicable to a manufacturing process/product created by another manufacturer. These process controls may also be confidential to the original manufacturer. Therefore, it would be difficult or impossible for a second manufacturer to make the "same" biologic without intimate knowledge of and experience with the innovator's process.

Further problems encountered by biological products derive from their natural variability. Various factors may compromise the consistency, safety and efficacy of these products; among them, special attention is to be given to the following:
a) All biological systems are inherently subject to genetic alteration through mutation and selection and foreign genes inserted into new host cells may exhibit increased genetic instability. The purpose of molecular genetic studies is to establish that the correct sequence has been made and incorporated in the host cell and that both the structure and the number of copies of the inserted sequence are maintained within the cell during culture to the end of production. Such studies can provide valuable information which should be considered in conjunction with tests performed at the protein level for assuring the quality and consistency of the product.
b) Products expressed in foreign hosts may deviate structurally, biologically or immunologically from their natural counterparts. Such alterations can arise at posttranslational level or during production or purification and may lead to undesirable clinical effects. Therefore, their presence must be justified and shown to be consistently controlled.
c) The choice of manufacturing procedure influences the nature, range and amount of potential impurities in the final product and which the purification processes must be shown to be capable of removing. Examples of these are endotoxins in products expressed in bacterial cells, and adventitious agents and DNA in products expressed in mammalian cells.
d) Unintended variability in the culture during production may lead to changes which favor the expression of other genes in the host/vector system or which cause alteration in the product. Such variation might result in differing yield, in change to the product itself (e.g. in the nature and degree of glycosylation) and/or in quantitative and qualitative differences in the impurities present. Consequently, in the field of biological products, procedures ensuring consistency of production conditions as well as the final product are imperative.
e) Extensive "scale-up" work, e.g. at the level of fermentation and/or purification, occurs as laboratory developments progress to full scale commercial production, and this may have considerable consequences for the quality of the product including effects on its conformational structure, yield and/or in quantitative and qualitative differences in impurities.

For these reasons the production of biologic products is typically a complex task, requiring extended in-process controls and quality control tests during each production run.

In alternative to the biological synthesis, a fully chemical synthetic approach has been proposed for the production of a number of low-sized immunogenic peptides. For example, the publication Scientific Reports, 7(1), 2017, pp. 1-11 describes the synthesis of a prototypical immune system engaging peptide with size of 5 KDa (corresponding to about 40 amino acids). The publication Chembiochem, 19(5), 2018, pp. 459-469 discloses a method of solid-phase synthesis and chemoselective ligation, to obtain effector peptides of about 7 KDa that bind to the formyl peptide receptor and stimulate an immune response. The publication Frontiers in Bioengineering and Biotechnology 8(1), 2020, p.162 describes the preparation, by solid phase synthesis and native chemical ligation, of sequence of more than 50 amino acids; the document points to the need to control the secondary and tertiary structure and refers to the synthesis of a 66-meric peptide of 7.3 KDa. None of these documents deals with the synthesis of scFv or similarly complex peptides. The patent application WO 99/55367 describes antibodies binding to the c-erbB2 receptor epitope; chemical synthesis is speculatively mentioned a possible preparation method, with reference to standard manuals for peptide synthesis, however no concrete evidence is given of the practicability of this method to generate the complex antibody structure, and no reproducible examples of synthesis are provided.

Accordingly, there is an unmet need of methods of fully chemical synthesis capable to generate functionally effective scFvs: these structures involve a significantly high number of amino acids, in the order of 200-300 amino acids. Particularly felt is the need to achieve via chemical synthesis an effective tertiary construction which reflects that of the biological reference product, i.e. being capable to reproduce its immunostimulant functionality: this result is not easily reachable by mere oxidation, especially for peptides dimensionally sized in the ranges mentioned herein. Moreover, there is also a need to develop production systems to make these molecules efficiently and cost effectively available; such new production systems would involve special challenges, particularly with respect to correct protein folding: only when a protein is correctly folded can it properly function. When a protein is manufactured its conformation may change in depending on its concentration, interaction partners, formulation buffer, etc.... Factors affecting protein to reach its final conformation could fail to create the correct hydrogen bonds changes the patterns needed to form these structures and negatively affects every other higher protein structure level. External factors such as temperature, pH, and formulation influence hydrogen bonding. Given that any structural change can impact functionality, the ability to accurately detect minimal changes in secondary and tertiary structure is critical to protein manufacturing and formulation. This is especially true for globular protein such as antibodies that are also named immunoglobulins for their globular aspect.

### SUMMARY OF THE INVENTION

The production of a scFv fragment using a fully chemical synthesis is described; this production system has the potential to become an alternative method for the rapid and highly parallel expression of a diverse range of antibody fragments. In comparison to traditional approaches where the scFv is obtained in biological systems, the present synthesis system can be time-saving, transforming a biologic in a chemically synthesized drug, releasing the production from the quality controls necessary for a biological product. Specifically, the method is directed to synthesize a scFv whose amino acid sequence (target sequence) contains 100 to 300, preferably 200 to 300 amino acids and comprises *n* cysteine amino acids (cysteine residues) separated by sub-sequences of *m* amino acids; the process comprises the steps of: (a) separately synthetizing all said sub-sequences, inclusive of their preceding cysteine residue if present, (b) sequentially assembling the sub-sequences obtained in step a. in the order as they appear in the target amino acid sequence, (c) performing oxidative folding of the assembled structure obtained in step b. and dialyzing the resulting product in a buffer.

### DESCRIPTION OF THE DRAWINGS

Figure 1: Primary structure and synthetic scheme of scFv Ab1 (D2B).

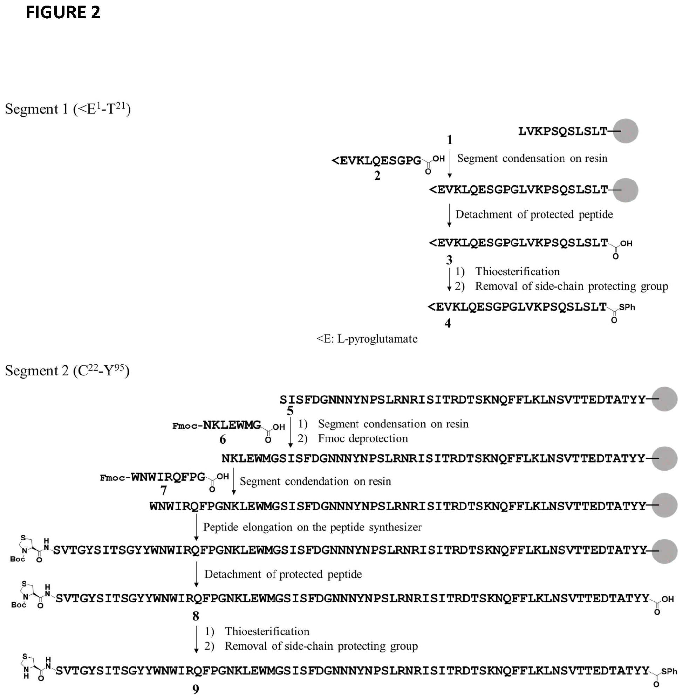

Figure 2: Synthetic routes for segments 1 and 2.

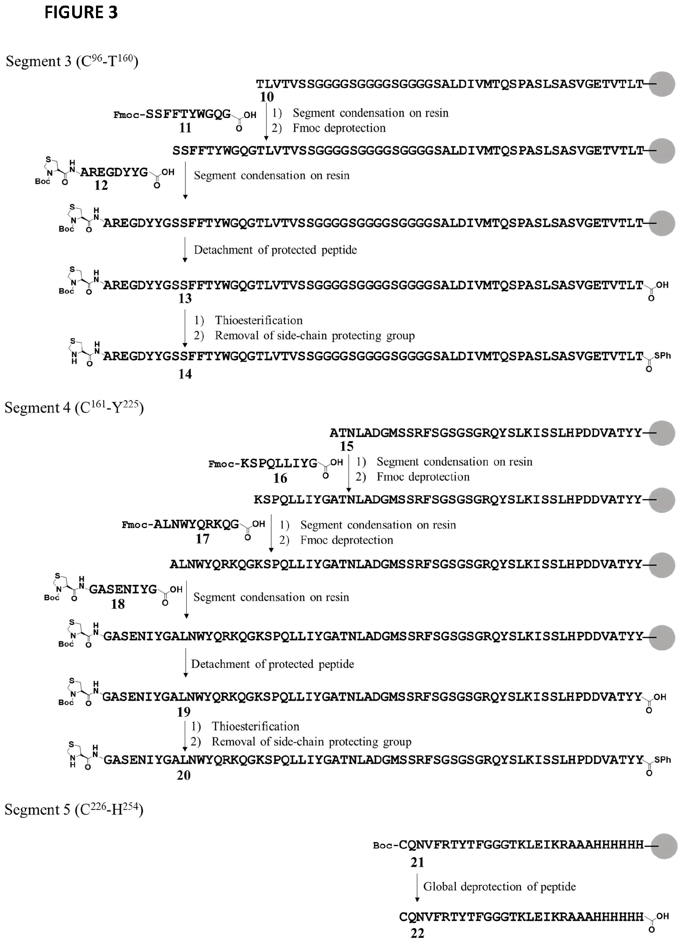

Figure 3: Synthetic routes for segments 3, 4 and 5.

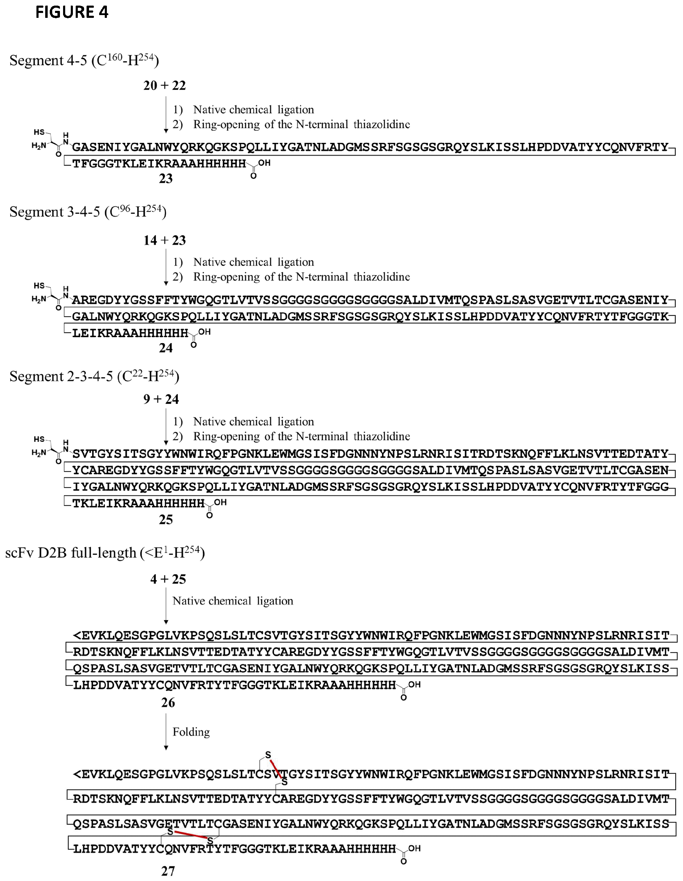

Figure 4. Assembly of the Ab1 (D2B) molecule through native chemical
   ligation followed by folding.
Figure 5: (A) HPLC elution profiles of the folding reaction of D2B. HPLC conditions: column, Imtakt Intrada WP-RP 3 µm (4.6 × 250 mm); gradient elution, 5-20% B (1 min)/20-35% (1-20 min); temperature, 80 °C; flow rate, 1.0 mL/min. a) Unfolded Ab1 (D2B) compound 26, b) one day after the folding reaction, c) purified folded Ab1 (D2B) compound 27. (B) ESI MS spectrum of folded D2B compound 27. ESI MS: m/z calcd for C₁₂₀₇H₁₈₂₃N₃₃₇O₃₈₁S₇ (27375.26) [M+10H]¹⁰⁺ 2738.53, [M+11H]¹¹⁺ 2489.66, [M+12H]¹²⁺ 2282.27; found 2738.43, 2489.34, 2282.19.
Figure 6: Binding of the scFv Ab3 evaluated in ELISA on human recombinant TNFα as positive antigen (red bar) and, on BSA as uncorrelated protein (orange bar) at concentration of 4.0 µg/ml. The scFv Ab3 of synthesis was resuspended and dialyzed in different ways: A) resuspended in water. B) resuspended in water and dialyzed in water. C) resuspended in selected buffer. D) resuspended in water and dialyzed in selected buffer. E) resuspended in water, dialyzed in water and dialyzed in selected buffer. The scFv Ab3 RREF was used as reference.
Figure 7: SDS-PAGE on acrylamide gel 4-12% Samples: A) ScFv Ab1; B) ScFv Ab2 and C) Ab3. Samples: 1) ScFvs of synthesis 2) ScFvs RREF M) Marker.
Figure 8: SEC Chromatogram performed with A) ScFv Ab1 of synthesis B) ScFv Ab1 RREF C) ScFv Ab2 of synthesis D) ScFv Ab2 RREF E) ScFv Ab3 of synthesis F) ScFv Ab3 RREF.
Figure 9: FACS analysis was performed on positive and negative cells. A) Reactivity of scFv Ab1 on: 1) positive (PC3-PIP) and 2) negative (HCC1937) cell line. B) Reactivity of scFv Ab2 on: 1) positive (MDAMB361) and 2) the negative (MDAMB468) cell line.
Figure 10: Binding, evaluated in ELISA at different concentrations, of the scFv A) scFv Ab1 on PC3-PIP (positive cells) and PC3 WT (negative cells). B) scFv Ab2 on MDAMB361 (positive cells) and MDAMB468 (negative cells). C) scFv Ab3 on human recombinant TNFα (positive antigen) and BSA (uncorrelated protein).
Figure 11: Sensorgrams from a Kinetics analyses of: (A) scFv Ab1 of synthesis or (B) scFv Ab1 RREF sample injections from 50 to 3.2 nM; (C) scFv Ab2 of synthesis or (D) scFV AB2 RREF sample injections from 12.5 to 0.8 nM; Sensorgrams from a Kinetics analyses of the purified (E) scFv Ab3 of synthesis or (F) scFV Ab3 RREF sample injections from 100 to 12.5 nM.

### DETAILED DESCRIPTION OF THE INVENTION

The method of the present invention relies entirely on chemical synthesis methods, i.e. it does not require biological steps (such as those using microorganisms, plant/animal cells or parts thereof), nor uses recombinant technologies. The method is directed to obtain scFv antibody fragments of a desired amino acid sequence (target sequence), which is the known sequence of the biological reference product, i.e the naturally occurring or biologically produced scFv. The method is applicable to the production of scFv containing a defined number of amino-acids. Particularly advantageous and unexpected is the efficacy of the present method to obtain target sequences in the range of 100 to 300 amino acids, or, more specifically, 150 to 200, or 150 to 250, or 150 to 300, or 200 to 300 or 250 to 300, or 230 to 280, corresponding to spatially complex structures. The method however also applies to the synthesis of target sequences containing a lesser number of amino acids, such as 100 to 150, 100 to 200, etc.

Compared to their biological reference product (RREF), the scFv produced by the present method share the binding specificity and display substantially identical binding kinetics. This functional equivalence is an indication that the present method is suitable to reproduce the primary structure (amino acid sequence) and the secondary and tertiary structure (three-dimensional folding) of the biological reference product. This allows to use the scFv obtained by the present invention for substantially the same uses/functions of the reference biological scFv. The correct folding of the scFv in the secondary and tertiary structure is generally caused by the formation of disulfide bonds between cysteine residues present in the primary structure, obtained by contacting the primary-structured peptide with a suitable folding buffer system, followed by treating the resulting product in the further defined conditions.

The target sequence of the scFv object of the present method comprises one or more cysteine amino acids (cysteine residues) variably spread along the sequence; they are preferably internal to the sequence, although they may be also present at one or both ends of the peptide as terminal amino acids. Accordingly, the target sequence is herein defined as a linear amino acid sequence comprising *n* cysteine residues separated by sub-sequences of *m* amino acids; *m* and *n* range within limits typical of scFv: in particular, *n* can be 1-10, preferably 2-6; *m* can be 0-100, preferably 5-80; the case m=0 correspond to a peptide wherein two adjacent cysteine amino acids are present, which is also comprised by the present invention. The above values of *m* and *n* are chosen under the condition that the number of amino acids of the resulting target sequence is comprised in one or more of the above defined sub-ranges.

The present method comprises the following general steps:
a. Separately synthetizing all said sub-sequences, inclusive of their preceding cysteine residue if present,
b. Sequentially assembling the sub-sequences obtained in step a. in the order as they appear in the target amino acid sequence,
c. Performing oxidative folding of the assembled structure obtained in step b. and dialyzing the resulting product in a buffer.

Step a. is composed of independent synthetic sub-steps, in which each of the sub-sequences of the target sequence are respectively produced. Each of these steps, independently from each other, can be performed via manual or automated peptide synthesis.

The manual synthesis of a sub-sequence may advantageously start from complementary fragments of the same, which are currently available or easily preparable; these fragments are subjected to a condensation reaction, with formation of an amide (peptide) bond between the free carboxy and amino groups of the respective amino acids to be bound; the selectivity of condensation is obtained by previously protecting the functional groups of the fragment not involved in the bonding: protecting groups/reactions are not limited and are conveniently chosen among those currently available in the art. For example, free amine groups may be protected via Fmoc (9-fluorenylmethyloxycarbonyl) derivatization; free carboxy groups may be protected via formation of suitable ester groups e.g. with trityl alcohol or benzyl alcohol. In addition, when glutamic acid or glutamine are present as N-terminal amino acids of the fragment, they may be respectively converted to pyroglutamate or pyroglutamine by conventional ring-closure reactions; restoration of the original amino acids can be obtained subsequently by conventional ring-opening reactions. Moreover, with exception of alanine, phenylalanine, glycine, isoleucine, leucine, methionine, proline and valine, the 20 natural amino acids have functional groups on their side chains, which also must not co-react and require protection: suitable protecting groups can be conveniently chosen as available in the art: for example, t-butyl protection is suitable for Asp, Glu, Ser, Thr and Tyr; t-butyloxycarbonyl (Boc) is suitable for Lys and Trp; 2,2,4,6,7-pentamethyldihydrobenzo-furan-5-sulfonyl (Pbf) is suitable for Arg; trityl is suitable for Asn, Cys, Gln and His; 3-methyl-3-pentyl (Epe) is suitable for Asp.

Preferably, the condensation reaction is performed on a resin (e.g. HMPB-Chem Matrix) on which a first fragment, protected on the groups not involved in the condensation reaction, is bound; the resin/fragment bonding reaction may conveniently involve the free carboxyl group to be protected, whereby the bonding reaction serves the double purpose of bonding and protecting; subsequently, a second fragment, protected on the groups not involved in the condensation, is eluted on the resin whereon the condensation reaction with the first fragment takes place; a resin-bound elongated fragment is thus obtained; further segment additions on the (suitably deprotected) amino terminal of the resin-bound peptide are possible, via the same sequence of steps described above; the last-added segment contains, as N-terminal amino acid, the cysteine residue which marks the start of the sub-sequence at hand; said cysteine residue is also present in protected form (e.g. in a cyclized thiazolidine form). Subsequently, the resin-bound complete sub-sequence is released and recovered: this is obtained by eluting the resin with a suitable solvent at suitable pH (e.g. 1.0% TFA in DCM), whereby the peptide/resin ester bond is hydrolyzed and the sub-sequence is released and collected; the carboxyl group formerly bound to the resin, now in free form, is subjected to thioesterification (e.g. by conversion to phenyl thioester); finally, the remaining protecting groups are removed.

The automated synthesis can follow the same steps referred above, including the relevant protection/deprotection reactions, being in this case performed on suitable peptide synthesizers (e.g. Protein Technologies Inc. AZ, USA),
In step b. the sub-sequences obtained in the step a are assembled, i.e. linearly connected, in the order as they appear in the target sequence. The sub-sequences to be connected obtained from step a. include their initial cysteine amino acid residue whose amino group, with exception of the first sub-sequence to be bound, is in protected form; the carboxyl terminal group of the sub-sequence, as obtained from step a. is in thioester form. The step b. is performed by native chemical ligation, followed by restoration of the original cysteine residue from its protected form, typically by reaction with an amine such as methoxamine. The native chemical ligation reaction proceeds in the presence of an arylthiol catalyst to chemoselectively and regioselectively produce a thioester-linked intermediate; the intermediate is rapidly and spontaneously rearranged by intramolecular S,N-acyl transfer to form a native amide bond at the ligation site (Dowson, P. E., Muir, T. W., Clark-Lewis, I., Kent, S. B. H. Science, 1994, 266, 776.)

Accordingly, the native chemical ligation of step b. comprises the following sub-steps: (a) dissolving the sub-sequences to be ligated in a ligation buffer, (b) stirring overnight, (c) stopping the reaction, (d) correcting the final pH to a range of 3-5, (e) purifying the resulting mixture. The ligation buffer used in (a) is preferably a phosphate buffer of pH 7 to which is added a mixture of mercaptophenylacetic acid, guanidine hydrochloride, tris(2-carboxyethyl)phosphine hydrochloride, and ascorbic acid or salt thereof; in (b) the stirring is preferably performed at room temperature; in (c) the stopping is preferably performed by addition of a mercaptoalkanesulphonate, optionally followed by addition of a hydroxylamine; in (d) the pH correction is preferably performed by addition of HCl; in (e) the purification is preferably performed by HPLC.

In step c., the oxidative folding is performed by adding the peptide to be folded in a folding buffer, followed by stirring for 1 day at room temperature and purifying the resulting product. The folding buffer is preferably a Tris buffer added with trehalose and guanidine hydrochloride, having final pH in the range 7-9. The resulting scFv is then dialyzed in a buffer, i.e. an aqueous buffered system; this passage was found important to guarantee the necessary level of binding specificity in the recovered scFv; in fact, a mere resuspension of the oxidative folding reaction mixture in an aqueous system, be it water or an aqueous buffer, or even a dialysis solely performed in water, were not capable to deliver a product with sufficient binding specificity;

Preferably the used buffer maintains the pH of the dialysis solution in the range of 4.6 to 8.0, preferably at 7.4 ± 0.5 as obtained e.g. by a phosphate-based buffer; alternative buffers can be used, e.g. based on acetate, citrate, glycine, histidine, etc. In addition to the buffer, the dialysis solution may contain substances commonly used in suspensions of antibodies, for example: (i) salts (e.g. sodium chloride or potassium chloride), preferably at concentration of 3-200 mM; (ii) chelating agents (e.g. EDTA) preferably at concentration of 1-15 mM; (iii) amino-acids (e.g. glycine, glutamate, methionine, proline), preferably at concentration of 1-300 mM; (iv) sugars (e.g. maltose, mannitol, sorbitol, sucrose trehalose), preferably at concentration of 12-300 mM; (iv) detergents (such as polysorbates), preferably at concentration of 0.1-1.6 wt %. After (or preferably before) said dialysis in buffer, the product subjected to oxidative folding may undergo a further dialysis, in water, i.e. pure, non-buffered water.

The techniques applied for the dialysis (apparatuses, working conditions, etc.) are per se known in the art (cf. Practical Skills in Biomolecular Sciences, 3r ed. Essex: Pearson Education Limited. p. 379. ISBN 978-0-13-239115-3) and are relied upon for the purpose of the present invention; details of the dialyzing step are included in the experimental section.

Preferred non-limitative applications of the method are directed to the sequence of D2B, herein abbreviated Ab1 (SEQ.ID.NO:1), trastuzumab, herein abbreviated Ab2 (SEQ.ID.NO:2) or adalimumab, herein abbreviated Ab3 (SEQ.ID.NO:3). Compared to the corresponding biologically-obtained scFv (references, RREF), the products obtained by the invention have the advantage of being free of biological contaminants (virus, endotoxins, etc.), i.e. they can be obtained with higher biological purity and do not involve the complex steps of biological purification; moreover, they provide a better inter-batch reproducibility due to the easier standardization of the chemical synthesis steps compared to biological ones.

The scFv obtained by the present method can be used in therapy as such, for the same immunologic application of the reference biological product. Alternatively, they can be used as starting materials for the preparation, via conventional methods, of complete antibodies, inclusive of their conventionally linked variable and constant portions.

The invention is now described in more detail by means of the following non-limitative application examples. The methods presented here are shown for mere exemplificative purpose and can be freely modified for the production of these or different products within the scope of the present invention.

### EXPERIMENTALS

Here we report the synthesis procedure of scFv and evidence of its activity being of the same order of the corresponding scFv of biological origin, obtained in prokaryotic system (reference biological scFv abbreviated RREF). The following scFv candidate was used as model:
**Ab1:** SEQ.ID NO.: 1 - **D2B:** a murine MAb belonging to the IgG1 and the variable domains belong for the VH to the murine mVH3 subgroup and for the light chain (VK) to the murine VK 12, 13 subgroups.
**Ab2:** SEQ.ID NO.: 2 - **Herceptin (Trastuzumab):** recombinant humanized antibody belonging to the IgG1 human isotype. The variable domains belong for VH to the human hVH3 subgroup and for the light chain (VK) to the human VK 1 subgroup.
**Ab3:** SEQ.ID NO.: 3 - **Humira (Adalimumab):** the first approved antibody selected from a phage display library to be approved by FDA is a recombinant human antibody belonging to the IgG1 human isotype. The variable domains belong for VH to the human hVH3 subgroup and for the light chain (VK) to the human VK 1 subgroup.

### 1. Synthesis of the scFv

This Example describes in detail the synthesis of the scFv of SEQ.ID.NO: 1 (D2B) for the purpose of exemplification; however, the same techniques and reagents were used to manufacture other scFv sequences, in particular those of SEQ.ID.NO: 2 (Trastuzumab) and SEQ.ID.NO: 3 (Adalimumab), obtained and successfully tested in the experimental section for equivalence with the corresponding biologically-obtained reference product. In particular, the skilled person will recognize that, within the limits of peptide sizes (number of amino acids) referred in the present description, these techniques and reagents are not peculiar of the exemplified scFv but are applicable to different scFvs.

### Abbreviations

Boc, *t*-butoxycarbonyl; *t*Bu, *t*-butyl; DCM, dichloromethane; DEPBT, 3-(diethoxyphosphoryloxy)-1,2,3-benzotriazin-4(3*H*)-one; DIC, *N,N'*-diisopropyl carbodiimide; DIPEA, diisopropylethylamine; DMF, *N,N*-dimethylformamide; DMS, dimethyl sulfide; EDT, 1,2-ethanedithiol; Epe, 3-methyl-3-pentyl; ESI MS, electrospray ionization mass spectrometry; Et₂O, diethyl ether; Fmoc, 9-fluorenylmethyloxycarbonyl; GnHCl, guanidine hydrochloride; HCTU, 1-[bis(dimethylamino)methylene]-5-chlorobenzotriazolium 3-oxide hexafluorophosphate; HMPB, 4-(4-hydroxymethyl-3-methoxyphenoxy)butyryl; HPLC, high performance liquid chromatography; MESNa, sodium 2-mercaptoethane sulfonate; MPAA, 4-mercaptophenylacetic acid; MSNT, 1-(mesitylene-2-sulfonyl)-3-nitro-1*H-*1,2,4-triazole; NMP, *N*-methyl-2-pyrrolidone; Oxyma pure, ethyl 2-cyano-2-(hydroxyimino)acetate; Pbf, 2,2,4,6,7-pentamethyldihydrobenzofuran-5-sulfonyl; PyBOP, (benzotriazol-1-yloxy)tripyrrolidinophosphonium hexafluorophosphate; RP-HPLC, reversed phase HPLC; SPPS, solid-phase peptide synthesis; TCEP, tris(2-carboxyethyl)phosphine hydrochloride; TFA, trifluoroacetic acid; Thz, thiazolidine-4-carboxylic acid; TIS, triisopropylsilane; Trt, trityl.

### Analysis and purification

Preparative HPLC was carried out on a Shimadzu Prominence LC-20AD HPLC system (Shimadzu Corp., Kyoto, Japan) with an Osaka Soda Capcell Pak C₁₈ UG120 5 µm (20 × 250 mm) column, a Waters Xselect CSH C₁₈ 5 µm (20 × 250 mm) column and an Agilent ZORBAX 300SB-CN 7 µm (21.2 × 250 mm) column at flow rate of 10 mL/min using a binary mixture of A (0.1% TFA in H₂O) and B [CH₃CN/H₂O/TFA (v/v, 90/10/0.09)] eluents. Separation was performed using the described linear gradient and detection at 220 nm. Analytical HPLC was performed on a Shimadzu Prominence LC-20AD HPLC system with an Imtakt Intrada WP-RP 3 µm (4.6 × 250 mm) column using a binary mixture of A and B eluents. The analysis was performed using a linear gradient at a flow rate of 1.0 mL/min and detection at 220 nm. ESI MS experiments were conducted on a Synapt HDMS mass spectrometer (Waters, MA, USA).

### Chemical synthesis rationale of scFv D2B

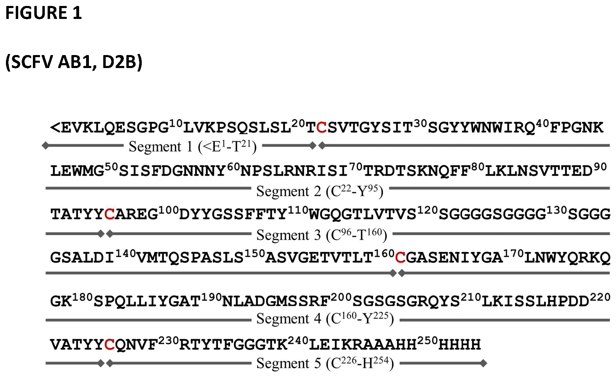

scFv D2B is comprised of 254 amino acid residues and contains two disulfide bonds (C²²-C⁹⁶ and C¹⁶¹-C²²⁶). The whole molecule was divided by cysteine residues into five segments and was assembled through native chemical ligation as shown in Figure 1. The resulting reduced molecule was subsequently subjected to the oxidative folding reaction under denatured conditions to form the complete scFv D2B molecule.

### Loading of the C-terminal Fmoc amino acid onto resin

Fmoc-amino acid was loaded onto HMPB-ChemMatrix resin and used for peptide elongation as follows: the *C*-terminal amino acid was introduced to HMPB-ChemMatrix resin (0.45 mmol/g) using the standard loading procedure with Fmoc-AA/1-(mesitylene-2-sulfonyl)-3-nitro-1*H*-1,2,4-triazole (MSNT)/*N*-methylimidazole (5/5/5 equiv.) in CH₂Cl₂ for 3 h. Each peptide segment having glycine at the *C*-terminus, namely **2**, **6**, **7**, **11**, **12**, **17** and **18**, was prepared in the form of a fully protected peptide with a free α-carboxy group, which was used for segment condensation on a solid support. Thus, for these segments Fmoc-Gly-OH was loaded onto 2-chlorotrityl resin (1.7 mmol/g, 100-200 mesh, 1% DVB) using Fmoc-Gly-OH/*N,N-*diisopropylethylamine (DIPEA) (0.7/4 equiv. relative to the resin functionality) in CH₂Cl₂ for 4 h, followed by washing the resin with CH₂Cl₂/MeOH/DIPEA (17:2:1), DMF and CH₂Cl₂.

### Automated peptide synthesis

Fmoc solid phase peptide synthesis (SPPS) by automated peptide synthesis was carried out on a Prelude automated peptide synthesizer (Protein Technologies, Inc., AZ, USA). The peptide chain was elongated using the standard Fmoc protocol of coupling with Fmoc-amino acid (except for Cys derivatives)/1-[*bis*(dimethylamino)-methylene]-5-chloro-1*H*-benzotriazolium 3-oxide hexafluorophosphate (HCTU)/ DIPEA (5.3/5.0/10 equiv.) or Fmoc-Cys(Trt)/HCTU/2,3,6-trimethylpyridine (5.3/5.0/20 equiv.) in *N,N-*dimethylformamide (DMF) (15 min). To improve and suppress the incomplete solvation of the peptide-resin complex during the peptide elongation, the following sequences were incorporated using the respective pseudoproline dipeptide units: Ile⁵²-Ser⁵³, Asp⁷³-Thr⁷⁴, Asp⁹⁰-Thr⁹¹, Val¹¹⁷-Thr¹¹⁸, Gly¹³⁵-Ser¹³⁶, Val¹⁵⁷-Thr¹⁵⁸, Ser¹⁹⁷-Ser¹⁹⁸, Tyr²⁰⁹-Ser²¹⁰ and Ala²²²-Thr²²³. Deprotection of *N*^{α}-Fmoc groups was performed using 20% piperidine/DMF (5 min × 2). During peptide synthesis, all washings after couplings and deprotections were performed with DMF. The following side chain-protecting groups were employed: *t*-butyl (*t*Bu) for Asp, Glu, Ser, Thr and Tyr, *t*-butyloxycarbonyl (Boc) for Lys and Trp, 2,2,4,6,7 pentamethyldihydrobenzo-furan-5-sulfonyl (Pbf) for Arg, trityl (Trt) for Asn, Cys, Gln and His, and 3-methyl-3-pentyl (Epe) for Asp^{55, 194, 219}, which is known to effectively reduce the aspartimide side reaction arising from the repetitive base treatment with 20% piperidine/DMF.

### Manual synthesis

Protected segments **2, 6, 7, 11, 12, 16, 17** and **18** used for convergent solid phase peptide synthesis (CSPPS) through segment condensation on solid support were manually synthesized using Fmoc-Gly loaded 2-chlorotrityl resin at a scale of 4 mmol. Peptide elongation was performed using the protocol of coupling with Fmoc-AA, Boc-Thz-OH or L-pyroglutamic acid (<E)/Oxyma pure/ *N,N'*-diisopropylcarbodiimide (5/5/5 equiv.) in NMP at room temperature for 2 h. Deprotection was performed using 20% piperidine/DMF (5 min ×2). Each protected segment was detached from the resin by treatment with 1% TFA in CH₂Cl₂ (20 mL, 5 min ×5) and all filtrates were collected in pyridine (5 mL). The solvent was removed under reduced pressure in a rotary evaporator. The residue was precipitated from 2% aqueous NaHCO₃, and the precipitate was washed with water and then lyophilized from water to give the crude product in the form of a fully protected peptide with a free α-carboxy group. Each crude product was used in the next reaction without further purification.

### Figure 2

### Compound 3

Protected peptide **1** on the resin, which was prepared with the peptide synthesizer in a 0.20 mmol scale, was treated with 20% piperidine in DMF (5 min ×2) to remove the *N-*terminal Fmoc group. After washing with DMF and CH₂Cl₂, it was coupled with protected peptide segment **2** (0.36 g, 0.24 mmol) in NMP (8.0 mL) in the presence of 3-(diethoxyphosphoryloxy)-1,2,3-benzotriazin-4(3*H*)-one (DEPBT, 0.14 g, 0.48 mmol) and DIPEA (0.12 mL, 0.72 mmol) at room temperature overnight. Protected segment **3** was detached from the resin by treatment with 1% TFA in CH₂Cl₂ (10 mL, 5 min ×14). All filtrates were collected in pyridine (5.0 mL) and the solvent was removed under reduced pressure in a rotary evaporator. The residue was precipitated from water, and the precipitate was washed with water and then lyophilized from water to give the crude protected peptide **3** (0.41 g).

### Compound 4

To a solution of the crude protected peptide **3** (0.41 g, 0.13 mmol) and thiophenol (0.41 mL, 3.8 mmol) in DMF (10 mL) was added PyBOP (0.33 g, 0.64 mmol) and DIPEA (0.11 mL, 0.64 mmol) sequentially at -15 °C, and the mixture was stirred overnight. The reaction was terminated by adding TFA (1.0 mL) at -15 °C and the mixture was then brought to room temperature. The solvent was removed under reduced pressure in a rotary evaporator at 40 °C. The resulting residue was treated with TFA/H₂O/triisopropylsilane (TIS)/dimethylsulfide (DMS) (v/v, 95:5:3:3) cleavage cocktail (8.0 mL) at room temperature. After 4 h, diethyl ether (Et₂O) was added to the reaction mixture to give a precipitate, which was washed with Et₂O and collected by centrifugation (× 10000 rpm, 10 min) twice before drying. Purification was carried out by preparative HPLC [column, Osaka Soda Capcell Pak C₁₈ UG-120 (20 × 250 mm); flow rate, 10 mL/min; temperature, 60 °C] using a gradient of 35-45% B in 30 min to give the peptide thioester **4 (segment 1 <E¹-Y⁹⁵)** (55 mg). ESI MS: *m*/*z* calcd for C₃₉₄H₅₇₅N₁₀₃O₁₁₈S₃ (2271.66): [M+2H]²⁺ 1136.83, [M+3H]³⁺ 758.22; found 1136.61, 758.07.

### Compound 8

The resin-bound protected peptide **5** (0.20 mmol) obtained after removal of the *N-*terminal Fmoc group was coupled with protected segment **6** (0.38 g, 0.24 mmol) in NMP (8.0 mL) in the presence of DEPBT (0.14 g, 0.48 mmol) and DIPEA (0.12 mL, 0.72 mmol) at room temperature overnight. The resulting peptide resin was treated with 20% piperidine in DMF to remove the *N*-terminal Fmoc group, which was then doubly coupled with protected segment **7** (0.57 g, 0.24 mmol) in NMP in the presence of DEPBT (0.14 g, 0.48 mmol) and DIPEA (0.12 mL, 0.72 mmol). The resulting D2B (35-95)-protected peptide resin was transferred to the automated synthesizer and peptide elongation was recommenced. After chain assembly of the D2B (22-95)-peptide was completed, protected segment **8** was detached from the resin by treatment with 1% TFA in CH₂Cl₂ (10 mL, 5 min ×20). All filtrates were collected in pyridine (10 mL) and the solvent was removed under reduced pressure in a rotary evaporator. The residue was precipitated from 2% aqueous NaHCO₃, and the precipitate was washed with water and then lyophilized from water to give the crude protected peptide 8 (1.8 g).

### Compound 9

To a solution of the crude protected peptide **8** (0.89 g, 61 µmol) and thiophenol (0.18 mL, 1.8 mmol) in DMF (10 mL) was added sequentially PyBOP (0.16 g, 0.31 mmol) and DIPEA (52 µL, 0.31 mmol) at -15 °C, and the mixture was stirred overnight. The reaction was terminated by adding TFA (0.50 mL) at -15 °C and the mixture was then brought to room temperature. The solvent was removed under reduced pressure in a rotary evaporator at 40 °C. The residue was treated with TFA/H₂O/TIS/DMS (v/v, 95:5:3:3) cleavage cocktail (16 mL) at room temperature. After 4 h, diethyl ether (Et₂O) was added to the reaction mixture to give a precipitate, which was washed with Et₂O and collected by centrifugation (×10000 rpm, 10 min) twice before drying. Purification was performed by preparative HPLC [column, Waters Xselect CSH C₁₈ 5 µm (20 x 250 mm); flow rate, 10 mL/min; temperature, 60 °C] using a gradient of 5-25% B (0-1 min)/25-50% B (1-20 min) to give the peptide thioester **9 (segment 2 C²²-Y⁹⁵)** (41 mg). ESI MS: *m*/*z* calcd for C₃₉₄H₅₇₅N₁₀₃O₁₁₈S₃ (8738.72): [M+4H]⁴⁺ 2185.48, [M+5H]⁵⁺ 1748.74, [M+6H]⁶⁺ 1457.45; found 2185.48, 1748.55, 1457.32.

### Figure 3

### Compound 13

The resin-bound protected peptide **10** (0.20 mmol) obtained after removal of the *N-*terminal Fmoc group was coupled with protected segment **11** (0.46 g, 0.24 mmol) in NMP (8.0 mL) in the presence of DEPBT (0.14 g, 0.48 mmol) and DIPEA (0.12 mL, 0.72 mmol) at room temperature overnight. The resulting peptide resin was treated with 20% piperidine in DMF to remove the *N*-terminal Fmoc group, which was then doubly coupled with protected segment **12** (0.39 g, 0.24 mmol) in NMP in the presence of DEPBT (0.14 g, 0.48 mmol) and DIPEA (0.12 mL, 0.72 mmol). The protectd peptide segment 3 [D2B (96-160)] was detached from the resin by treatment with 1% TFA in CH₂Cl₂ (10 mL, 5 min x20). All filtrates were collected in pyridine (5.0 mL) and the solvent was removed under reduced pressure in a rotary evaporator. The residue was precipitated from 2% aqueous NaHCO₃, and the precipitate was washed with water and then lyophilized from water to give the crude protected peptide **13** (0.30 g).

### Compound 14

To a solution of the crude protected peptide **13** (0.47 g, 55 µmol) and thiophenol (0.18 mL, 1.7 mmol) in DMF (15 mL) was added sequentially PyBOP (0.14 g, 0.28 mmol) and DIPEA (48 µL, 0.28 mmol) at -15 °C, and the reaction mixture was stirred overnight. The reaction was terminated by adding TFA (0.10 mL) at -15 °C and the mixture was then brought to room temperature. The solvent was removed under reduced pressure in a rotary evaporator at 40 °C. The residue was treated with TFA/H₂O/TIS/DMS (v/v, 95:5:3:3) cleavage cocktail (16 mL) at room temperature. After 4 h, Et₂O was added to the reaction mixture to give a precipitate, which was washed with Et₂O and collected by centrifugation (×10000 rpm, 10 min) twice before drying. Purification was performed by preparative HPLC [column, Agilent ZORBAX 300SB-CN 7 µm (21.2 × 250 mm); flow rate, 10 mL/min; temperature, 80 °C] using a gradient of 5-30% B (0-1 min)/30-47.4% B (1-20 min) to give the peptide thioester **14 (segment 3 C⁹⁶-T¹⁶⁰)** (46 mg). ESI MS: *m*/*z* calcd for C₂₇₂H₄₀₉N₇₁O₉₆S₃ (6305.85) [M+3H]³⁺ 2102.95, [M+4H]⁴⁺ 1577.46, [M+5H]⁵⁺ 1262.17; found 2102.89, 1577.17, 1262.14.

### Compound 19

The synthesis of protected **segment 4 (C¹⁶¹-Y²²⁵)** was performed by sequential assembly of protected segments **16** (0.79 g, 0.48 mmol) and **17** (1.3 g, 0.48 mmol) onto the resin-bound protected peptide **15** (0.40 mmol) using the DEPBT (0.29 g, 0.96 mmol)/DIPEA (0.25 mL, 1.4 mmol) method in NMP (8.0 mL) at room temperature overnight. The resin-bound protected D2B (170-225) obtained after removal of the *N*-terminal Fmoc group was doubly coupled with segment **18** (0.67 g, 0.48 mmol) using the same procedure as described above to give the resin-bound **segment 4 (C¹⁶¹-Y²²⁵)**]. The protected peptide segment **19** was detached from the resin by treatment with 1% TFA in CH₂Cl₂ (20 mL, 5 min ×15). All filtrates were collected in pyridine (10 mL) and the solvent was removed under reduced pressure in a rotary evaporator. The residue was precipitated from 2% aqueous NaHCO₃, and the precipitate was washed with water and then lyophilized from water to give the crude protected peptide **19** (2.7 g)

### Compound 20

To a solution of the crude protected peptide **19** (2.7 g, 0.24 mmol) and thiophenol (0.76 mL, 7.1 mmol) in DMF (15 mL) was added sequentially PyBOP (0.62 g, 1.2 mmol) and DIPEA (0.21 mL, 1.2 mmol) at -15 °C, and the mixture was stirred overnight. The reaction was terminated by adding TFA (1.0 mL) at -15 °C and the mixture was brought to room temperature. The solvent was removed under reduced pressure in a rotary evaporator at 40 °C. The residue was treated with TFA:H₂O:TIS:DMS (v/v, 95:5:3:3) cleavage cocktail (20 mL) at room temperature. After 4 h, Et₂O was added to the reaction mixture to give a precipitate, which was washed with Et₂O and collected by centrifugation (×10000 rpm, 10 min) twice before drying. Purification was performed by preparative HPLC [column, Waters Xselect CSH C18 5 µm (20 × 250 mm); flow rate, 10 mL/min; temperature, 60 °C] using a gradient of 5-25% B (0-1 min)/25-39.5% B (1-11 min) to give the peptide thioester **20 segment 4 (C¹⁶¹-Y²²⁵)** (99 mg). ESI MS: *m*/*z* calcd for C₃₁₈H₄₇₉N₈₇O₉₈S₃ (7185.02) [M+5H]⁵⁺ 1438.00, [M+6H]⁶⁺ 1198.50, [M+7H]⁷⁺ 1027.43; found 1438.02, 1198.53, 1027.48.

### Compound 22

The resin-bound protected peptide **21** (0.40 mmol) was treated with TFA:H₂O:TIS:DMS:1,2-ethanedithiol (EDT) (v/v, 95:5:3:3:2) cleavage cocktail (20 mL) at room temperature for 3 h. Et₂O was added to the reaction mixture to yield a precipitate, which was washed with Et₂O twice and dried. Purification was performed by preparative HPLC [column, Waters Xselect CSH C18 5 µm (20 × 250 mm); flow rate 10 mL/min; 60 °C] using a gradient of 5-15% B (0-1 min)/15-22% B (1-20 min) to give compound **22 segment 5 (C²²⁶-H²⁵⁴),** (86 mg). ESI MS: *m*/*z* calcd for C₁₄₈H₂₂₁N₅₁O₃₈S (3354.78) [M+3H]³⁺ 1119.26, [M+4H]⁴⁺ 839.70, [M+5H]⁵⁺ 671.96; found 1119.26, 839.70, 671.75.

### Figure 4

### Compound 23

Segment 5 (compound **22**, 58 mg, 17 µmol) and peptide thioester compound **20** (99 mg, 14 µmol) were dissolved in freshly prepared ligation buffer [0.2 M Na₂HPO₄ containing 50 mM 4-mercaptophenylacetic acid (MPAA) and 8 M guanidine hydrochloride (GnHCl), pH 7.8, 13 mL], and then 0.5 M tris(2-carboxyethyl)phosphine hydrochloride (TCEP)/H₂O (pH 7, 0.70 mL) and 0.5 M L-ascorbic acid sodium salt/H₂O (0.7 mL) were added sequentially to the peptide solution. After agitating the reaction mixture overnight at room temperature, the reaction was termin ated by adding sodium 2-mercaptoethanesulfonate (MESNa, 0.46 g, 2.8 mmol), 0.5 M L-ascorbic acid sodium salt/H₂O (0.70 mL) and 0.5 M TCEP/H₂O (pH 7, 0.70 mL). *O*-Methylhydroxylamine hydrochloride (0.13 g, 1.5 mmol) was added to the ligation mixture, and the mixture was then adjusted to pH 3.8 with 2 M HCl. After agitating the reaction mixture overnight at room temperature, the mixture was purified by preparative HPLC [column, Waters Xselect CSH C18 5 µm (20 × 250 mm); flow rate 10 mL/min; temperature, 60 °C] using a gradient of 5-20% B (0-1 min)/20-34.7% B (1-15 min) to give compound **23** (53 mg). ESI MS: *m*/*z* calcd for C₄₅₉H₆₉₄N₁₃₈O₁₃₆S₃ (10417.16) [M+10H]¹⁰⁺ 1042.76, [M+11H]¹¹⁺ 948.06, [M+12H]¹²⁺ 869.13; found 1042.68, 948.09, 869.14.

### Compound 24

Segment (4-5) compound **23** (53 mg, 5.1 µmol), and segment 3-thioester compound **14** (46 mg, 7.3 µmol) were dissolved in freshly prepared ligation buffer (0.2 M Na₂HPO₄ containing 50 mM MPAA and 8 M GnHCl, pH 7.8, 4.5 mL), and then 0.5 M TCEP/H₂O (pH 7, 0.25 mL) and 0.5 M L-ascorbic acid sodium salt/H₂O (0.25 mL) were added sequentially to the peptide solution. After agitating the reaction mixture for 1 day at room temperature, the reaction was terminated by adding MESNa (0.16 g, 1.0 mmol), 0.5 M L-ascorbic acid sodium salt/H₂O (0.25 mL) and 0.5 M TCEP/H₂O (pH 7, 0.25 mL). *O*-Methylhydroxylamine hydrochloride (0.13 g, 1.5 mmol) was added to the ligation mixture, and the mixture was then adjusted to pH 3.8 with 2 M HCl. After agitating the reaction mixture for 5 h, the mixture was purified by preparative HPLC [column, Imtakt Intrada WP-RP 3 µm (4.6 × 250 mm); flow rate 1.0 mL/min; temperature, 80 °C] using a gradient of 5-20% B (0-1 min)/20-29.5% B (1-9 min) to give the segment (3+4+5), compound **24** (35 mg). ESI MS: *m*/*z* calcd for C₇₂₄H₁₀₉₇N₂₀₉O₂₃₂S₅ (16601.27) [M+7H]⁷⁺ 2372.61, [M+8H]⁸⁺ 2076.16, [M+9H]⁹⁺ 1845.59; found 2372.35, 2076.18, 1845.46.

### Compound 25

To a solution of segment (3-4-5) compound **24** (35 mg, 2.1 µmol) and segment 2-thioester compound **9** (41 mg, 4.7 µmol) in freshly prepared ligation buffer (0.2 M Na₂HPO₄ containing 50 mM MPAA and 8 M GnHCl, pH 7.9, 1.8 mL) was added 0.5 M TCEP/H₂O (pH 7, 0.10 mL) and 0.5 M L-ascorbic acid sodium/H₂O (0.10 mL) sequentially. After agitating the reaction mixture at room temperature overnight, the reaction was terminated by adding MESNa (66 mg, 3.9 mmol), 0.5 M L-ascorbic acid sodium salt/H₂O (0.1 mL) and 0.5 M TCEP/H₂O (pH 7, 0.1 mL). *O-*Methylhydroxylamine hydrochloride (52 mg, 0.6 mmol) was added to the ligation mixture, and the mixture was then adjusted to pH 3.8 with 2 M HCl. The mixture was agitated at room temperature overnight and purified by HPLC [column, Imtakt Intrada WP-RP 3 µm (4.6 × 250 mm); flow rate 1.0 mL/min; 80 °C] using a gradient of 5-20% B (0-1 min)/20-27.1% B (1-10 min) to give the segment (2-3-4-5), compound **25** (7.0 mg). ESI MS: *m*/*z* calcd for C₁₁₁₁H₁₆₆₆N₃₁₂O₃₅₀S₇ (25217.80) [M+27H]²⁷⁺ 934.99, [M+28H]²⁸⁺ 901.64, [M+29H]²⁹⁺ 870.58; found 934.93, 901.46, 870.61.

### Compound 26

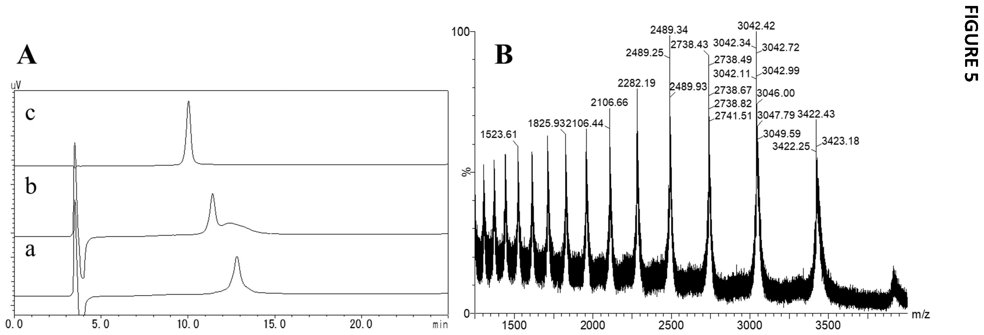

To a solution of segment (2-3-4-5) compound **25** (7.0 mg, 0.30 µmol) and segment 1-thioester **4** (2.0 mg, 0.60 µmol) in freshly prepared ligation buffer (0.2 M Na₂HPO₄ containing 50 mM MPAA and 8 M GnHCl, pH 7.9, 0.3 mL) was added 0.5 M TCEP/H₂O (pH 7, 15 µL) and 0.5 M L-ascorbic acid sodium salt/H₂O (15 µL) sequentially. After agitating the reaction mixture for 1 day at room temperature, the reaction was terminated by adding MESNa (2.0 mg, 12 µmol), 0.5 M L-ascorbic acid sodium salt/H₂O (15 µL) and 0.5 M TCEP/H₂O (pH 7, 15 µL). The reaction mixture was purified by HPLC [column, Imtakt Intrada WP-RP 3 µm (4.6 × 250 mm); flow rate, 1.0 mL/min; temperature, 80 °C] using a gradient of 5-20% B (0-1 min)/20-27.9% B (1-10 min) to give the segment (1-2-3-4-5), compound **26** (4.0 mg). Figure 5A (a)ESI MS: *m*/*z* calcd for C₁₂₀₇H₁₈₂₇N₃₃₇O₃₈₁S₇ (27379.29) [M+15H]¹⁵⁺ 1826.29, [M+16H]¹⁶⁺ 1712.21, [M+17H]¹⁷⁺ 1611.55; found 1826.26, 1712.47, 1611.46.

### Compound 27

Segment (1-2-3-4-5) compound **26** (4.0 mg, 0.15 µmol) was dissolved in folding buffer (0.1 M tris-HCl containing 0.2 M trehalose and 8 M GnHCl, pH 8.6, 4.0 mL) and the solution was agitated for 1 day at room temperature. The folding reaction mixture was then purified by RP-HPLC [column, Imtakt Intrada WP-RP 3 µm (4.6 × 250 mm); flow rate, 1.0 mL/min; temperature, 80 °C) using a gradient of 5-20% B (0-1 min)/20-26.3% B (1-9 min) to give the folded scFv D2B compound **27** (1.0 mg) figure 5A (c) . ESI MS: *m*/*z* calcd for C₁₂₀₇H₁₈₂₃N₃₃₇O₃₈₁S₇ (27375.26) [M+10H]¹⁰⁺ 2738.53, [M+11H]¹¹⁺ 2489.66, [M+12H]¹²⁺ 2282.27; found 2738.43, 2489.34, 2282.19. Figure 5B

### Protein concentration measurement

Folded protein ScFv full-length compound **27** (1.0 mg) was dissolved in water (1.0 mL) and the protein concentration (0.047 mM, 1.3 mg/mL) of the solution was determined by measuring its absorbance at 280 nm with a NanoDrop 2000c spectrophotometer using a calculated extinction coefficient of 51590 M⁻¹·cm⁻¹ and molecular weight of 27375.26. The solution was divided into five aliquots (200 µL each) followed by lyophilization, and four aliquots were used for assays, analyses and dialysed as described below.

### Dialysis

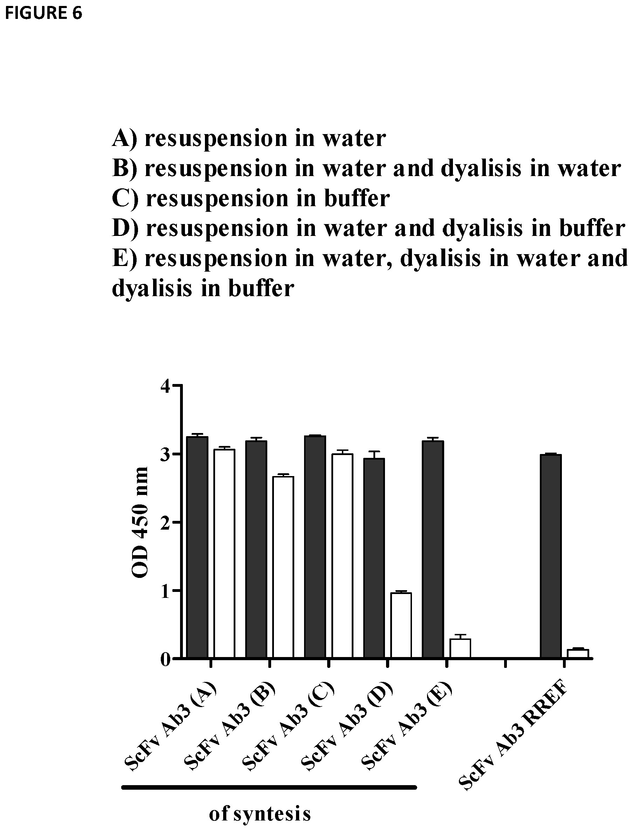

Two dialysis steps were performed (Practical Skills in Biomolecular Sciences, 3rd ed. Essex: Pearson Education Limited. p. 379. ISBN 978-0-13-239115-3). We utilized SpectraPor2 Dialysis membrane from Spectrumlabs (MCWO 12-14 KDa). The sample was resuspended in water and extensive dialyzed in 100x volume of water. A second process of dialysis in buffer was performed and the best buffer was 10 mM sodium hydrogen phosphate 150 mM sodium chloride, EDTA 3mM adjusted to pH 7.4. The dialysis in buffer was found necessary for correct folding of scFvs. In fact, as demonstrated by the data in figure 6 (ScFv SEQ.ID.NO.: 3), the binding of scoffs whether resuspended only in water or buffer, or even dialyzed in sole water, displayed a nonspecific binding, visible on BSA (uncorrelated protein). Equivalent results were obtained in this test using the scFvs Ab1 and Ab2.

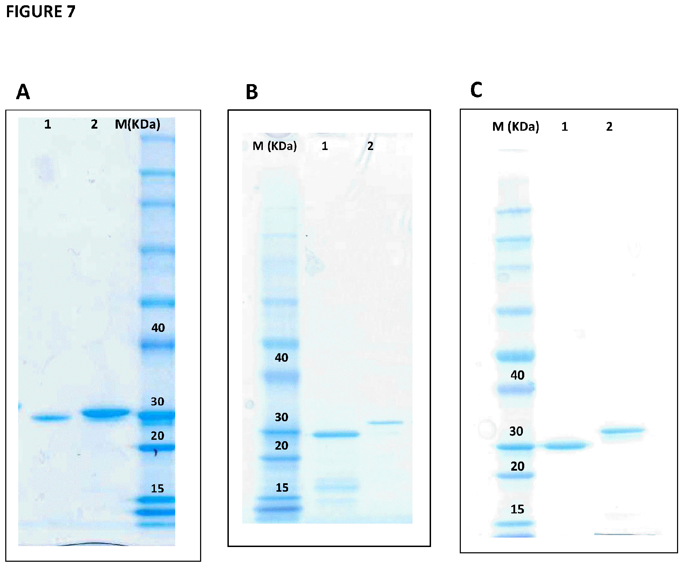

The scFvs produced by synthesis has the expected molecular weight of the biological reference product, as demonstrated in SDS-PAGE (Figure 7: A) scFv Ab1; B) scFv Ab2 and C) scFv Ab3) the small weigth differences between the two products is due to the fact that the scFvs RREF contain two tags (Myc and His) whereas in the synthesis product only the His tag is present.

### 2. Size Exclusion Chromatography testing

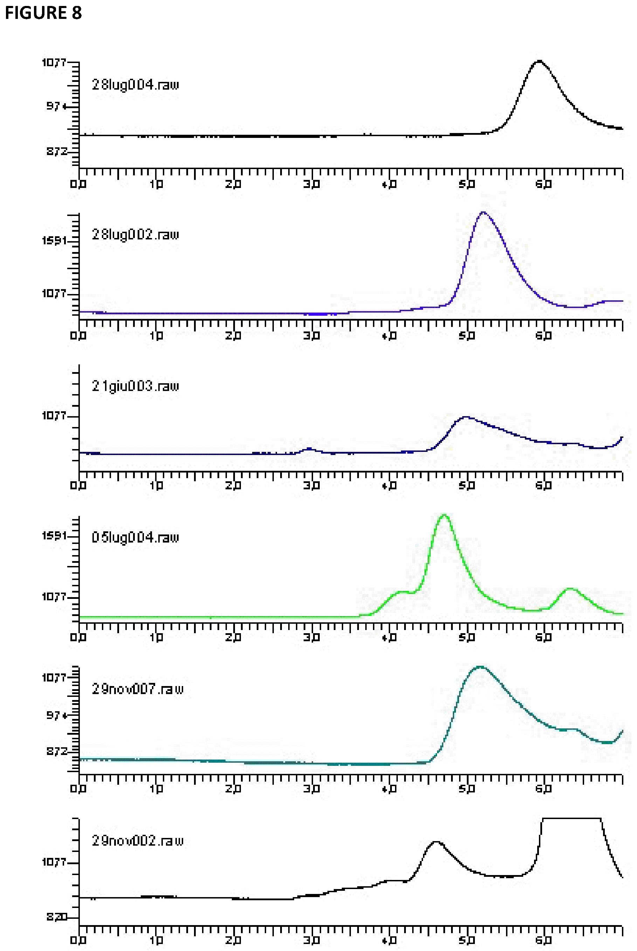

Size Exclusion Chromatography (SEC) is a well-established method to separate proteins and polymers by size and is the method of choice for the determination of aggregates in the quality control of biopharmaceuticals. In the SEC profile (Figure 8 A. scFv Ab1; B. scFv Ab2 and C. scFv Ab3) indicated that the scFvs produced by synthesis do not have aggregated and also the few amounts of dimers present in the ScFvs RREF are not present. Of note the scFv produced by synthesis is the only one that reached almost a homogeneity of 100%.

The measurements were performed on Series 200 HPLC system (Perkin Elmer) consisting of a solvent pump, an autosampler and a UV detector.

The data was analyzed with the TC Nav software. A Superdex 75 5/15 GL (15 x 5 mm) column (Cytiva) was used. A salt buffer was used consisting of 10 mM sodium hydrogen phosphate 150 mM sodium chloride, adjusted to pH 7.4. An injection volume of 10 µl, a flow rate of 0.3 ml/min and a detection wavelength of 280 nm were applied.

### 3. Functional analysis of scFv molecules

Antigen binding of synthesized scFv fragments was then analyzed by FACS \ELISA and Surface Plasmon Resonance using Biacore T200.

### FACS (Fluorescence Activated Cell Sorter) testing

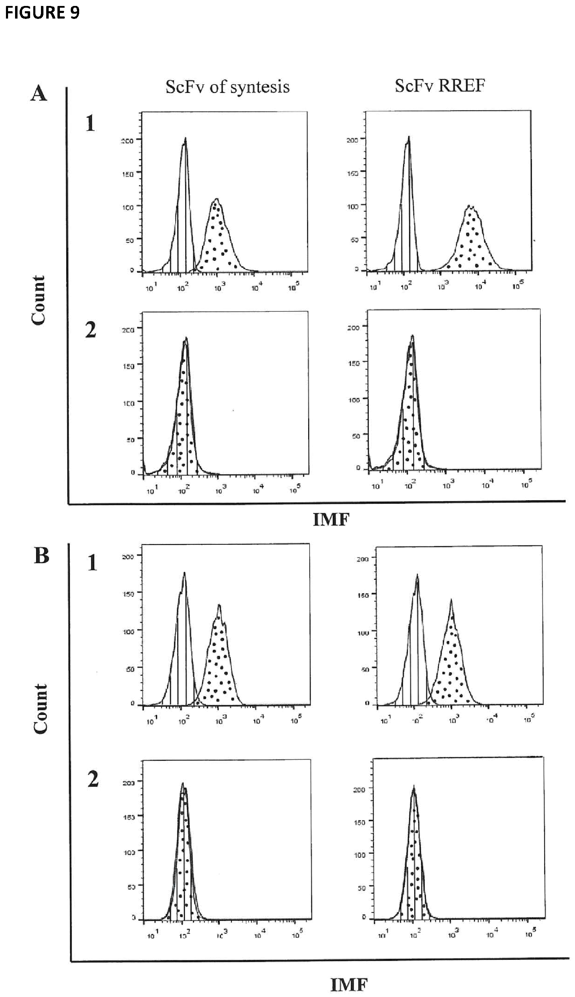

The scFvs produced by chemical synthesis or scFvs RREF produced in prokaryotic system (ScFv Ab1 SEQ.ID.NO.: 1; ScFv Ab2 SEQ.ID.NO.: 2) (10 µg/ml) were added to tumour cell (5x10⁵) in 100 µl PBS+ 0.03% BSA, and the mixture was incubated for 30 min on ice. The binding was detected by an antibody anti His Tag (product in mouse) for 30 min on ice. All antibodies binding was revealed with an antibody anti-mouse IgG-Alexa488 for 30 min on ice. For each sample 5000 cells were analysed with FACS Canto using DIVA software. The results are presented in Figure 9 (A scFv Ab1 and B scFv Ab2). All the tested antibodies show specificity for the positive antigen cells (Fig. 9A1 and Fig. 9B1) and negative cells that do not have the antigen (Fig. 9A2 and Fig. 9B2).

### ELISA (Enzyme-Linked Immunosorbent Assay) testing

For the test 5x10⁴ tumor cells were seed on a 96-weel plate and fixed by glutaraldehyde (0.1% in PBS for 5 min) and glycine (0.1 M in PBS for 10 min).

The plate was saturated for 2 hours with BSA 1% in PBS.

All the scFvs antibodies were tested in double in dilution from 2.5 to 0.0025 µg/ml. The plate was incubated for one hour at room temperature. The binding was detected by an antibody anti His Tag (product in mouse) for one hour at room temperature and subsequently with an HPR-conjugated antibody anti-mouse IgG. Develop of the test with 100 µl of 3,3',5,5'-tetrametilbenzidina (TMB) and read at 450 nm after blocking with 50 µl of sulfuric acid 1 M.

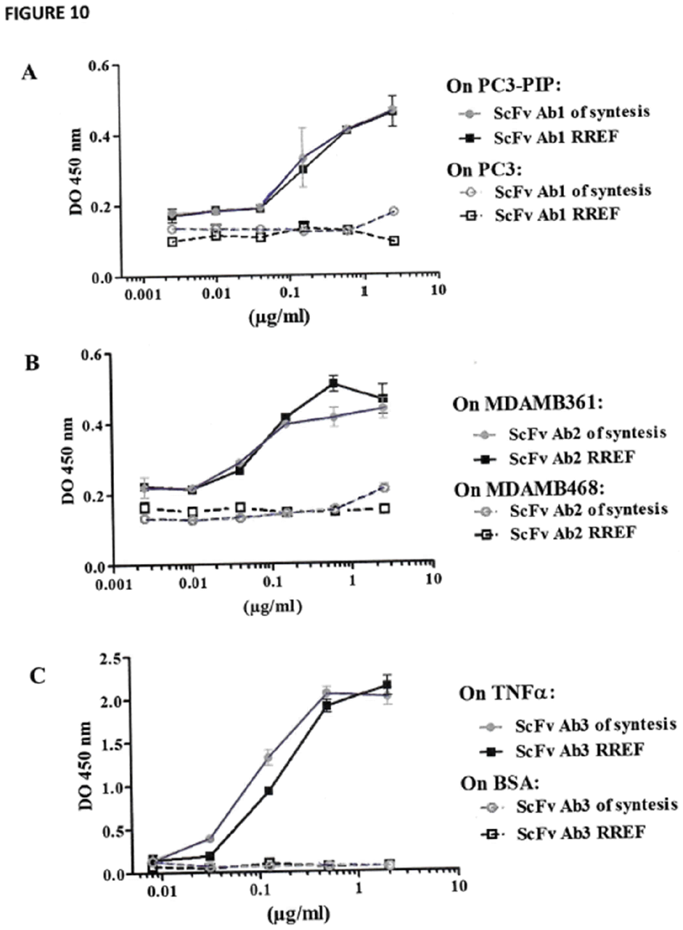

The results are reported in Figure 10A for scFv Ab1, Figure 10B for scFv Ab2, Figure 10C for scFv Ab3 showing the binding of the scFvs produced in different system on positive cells (respectively PC3-PIP for scFv Ab1 and MDAMB361 for scFv Ab2) or positive antigen (TNFα for scFv Ab3) and on negative cells (respectively PC3 for scFv Ab1 and MDAMB468 for scFv Ab2) or uncorrelated protein (BSA for scFv Ab3) at different concentrations. The graphics show that that the curves of the scFvs obtained by chemical synthesis presents substantially the same binding behavior, within the experimental deviation limits, of the product scFvs RREF; the same conclusion can be drawn for the set of curves relevant to negative cells or uncorrelated protein.

### Surface Plasmon Resonance testing using Biacore T200

Biacore is an instrument based on Surface Plasmon Resonance for the characterization of biomolecular interactions, in this case antibodies and antigen (Jason-Moller L et al Curr Protoc Protein Sci. 2006 Sep; Chapter 19: Unit 19.13).

The scFvs produced by synthesis and the scFv RREF were tested using the standard Kinetics method to measure the affinity of the scFv produced with different systems.

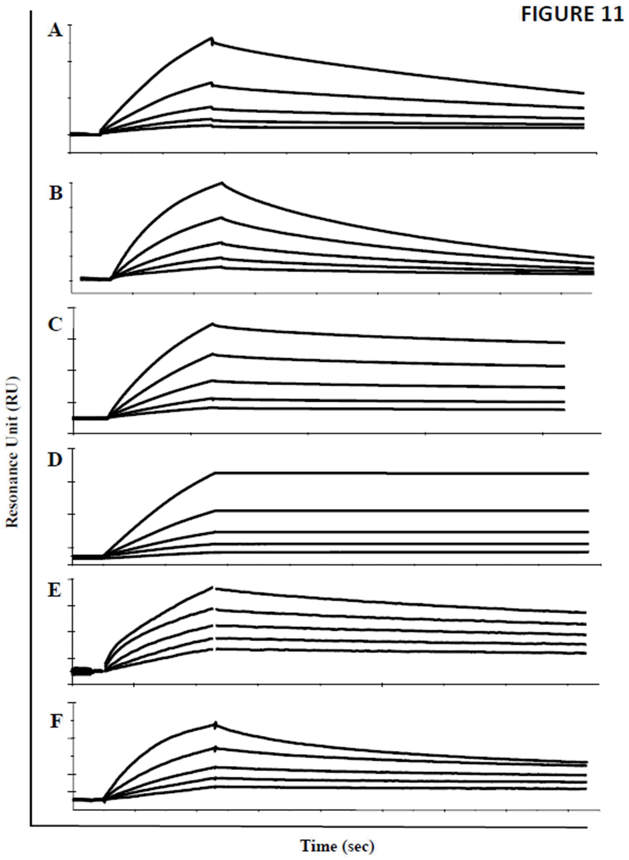

The standard Kinetics it was carried out immobilizing the purified and soluble antigen on sensorchip CM5 (respectively hPSMA for scFv Ab1; hHER2-ECD for scFv Ab2 and hTNFα for scFv Ab3) on the CMS sensorchip (Figure 11).

The results are reported in table I, II and III together with the Chi² and the pValue that indicate the statistical goodness of the experiments (Chi² < 10% of RU max and pValue < 16)

**Table I**

| scFv Ab 1 | **KD (nM)** | **RUₘₐₓ** | **Chi²** | **Pvalue** |
|---|---|---|---|---|
| synthesis | 12 | 5 | 0.02 | 4 |
| RREF | 27 | 6 | 0.03 | 3 |

**Table II**

| scFv Ab2 | **KD (nM)** | **RUₘₐₓ** | **Chi²** | **Pvalue** |
|---|---|---|---|---|
| syntesis | 0.33 | 343 | 3.3 | 1 |
| RREF | 0.03 | 824 | 8.2 | 12 |

**Table III**

| scFv Ab3 | **KD (nM)** | **RUₘₐₓ** | **Chi²** | **Pvalue** |
|---|---|---|---|---|
| syntesis | 4.3 | 12 | 0.4 | 2 |
| RREF | 9.3 | 19 | 0.4 | 2 |

The sensorgram curves of all the tested scFv, i.e. biologically vs. chemically synthetized according to the invention, consistently overlap, indicating equivalence of behavior.

In conclusion, the experimental results presented in in this experimental section show that the full-chemical synthesis of the scFvs is feasible and reliable, resulting in the same primary structure and substantial the same secondary structure of the reference biological product, substantially maintaining the same biological activity. The objectives of the invention are thereby reached.

### SEQUENCE LISTINGS

SEQ ID. NO.: 1 (scFv D2B) 254 aa 
EVKLQESGPGLVKPSQSLSLTCSVTGYSITSGYYWNWIRQFPGNKLEWMGSIS
FDGNNNYNPSLRNRISITRDTSKNQFFLKLNSVTTEDTATYYCAREGDYYGSS
FFTYWGQGTLVTVSSGGGGSGGGGSGGGGSALDIVMTQSPASLSASVGETVT
LTCGASENIYGALNWYQRKQGKSPQLLIYGATNLADGMSSRFSGSGSGRQYS
LKISSLHPDDVATYYCQNVFRTYTFGGGTKLEIKRAAAHHHHHH
SEQ ID. NO.: 2 (scFv Ab2; scFv Trastuzumab) 252 aa 
EVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYA
MDYWGQGTLVTVSSGGGGSGGGGSGGGGSDIQMTQSPSSLSASVGDRVTITCRA
SQDVNTAVAWYQQKPGKAPKLLIYSASFLYSGVPSRFSGSRSGTDFTLTISSLQPE
DFATYYCQQHYTTPPTFGQGTKVEIKRAAAHHHHHH
SEQ ID. NO.: 3 (scFv Ab3; scFv Adalimumab) 252 aa

EVQLVESGGGLVQPGRSLRLSCAASGFTFDDYAMHWVRQAPGKGLEWVSAI
TWNSGHIDYADSVEGRFTISRDNAKNSLYLQMNSLRAEDTAVYYCAKVSYLS
TASSLDYWGQGTLVTVSGGGGSGGGGSGGGGSDIQMTQSPSSLSASVGDRVT
ITCRASQGIRNYLAWYQQKPGKAPKLLIYAASTLQSGVPSRFSGSGSGTDFTLT
ISSLQPEDVATYYCQRYNRAPYTFGQGTKVEIKRAAAHHHHHH

## Claims

1. Method to synthesize a single-chain variable antibody fragment (scFv) whose amino acid sequence (target sequence) comprises *n* cysteine residues separated by sub-sequences of *m* amino acids, wherein said target sequence contains 100 to 300 amino acids, said process comprising the steps of:
a. Separately synthetizing all said sub-sequences, inclusive of their preceding cysteine residue if present,
b. Sequentially assembling the sub-sequences obtained in step a. in the order as they appear in the target amino acid sequence,
c. Performing oxidative folding of the assembled structure obtained in step b. and dialyzing the resulting scFv in a buffer.

2. Method according to claim 1, wherein n is 1-10, preferably 2-6, and m is 0-100, preferably 5-80.

3. Method according to claims 1-2, wherein said target sequence contains 200 to 300 amino acids.

4. Method according to claims 1-3, wherein the step a. is performable via manual or automated peptide synthesis.

5. Method according to claim 4, where wherein the manual peptide synthesis includes a condensation on resin and the automated peptide synthesis is a peptide elongation performed on a peptide synthesizer.

6. Method according to claims 1-5 where, in step a.: (a) the preceding cysteine residue is present as a N-protected precursor thereof, and (b) the final carboxy group of the obtained sub-sequence is further converted in thioester form.

7. Method according to claim 6, wherein said thioester is a phenyl thioester and said N-protected cysteine precursor is a N-protected cyclized cysteine.

8. Method according to claims 1-7, where in step b. said assembling is performed by native chemical ligation, followed by restoration of the original cysteine residue, if the same is present as a N-protected precursor thereof.

9. Method according to claims 1-8, where said native chemical ligation comprises: (a) dissolving the sub-sequences to be ligated in a ligation buffer, (b) stirring overnight, (c) stopping the reaction, (d) bringing the pH of the reaction mixture to a range of 3-5, (e) purifying the resulting mixture.

10. Method according to claim 9, wherein: in (a) the ligation buffer is a phosphate buffer of pH 7 to which is added a mixture of mercaptophenylacetic acid, guanidine hydrochloride, tris(2-carboxyethyl)phosphine hydrochloride, and ascorbic acid or its salt thereof; in (c) the stopping is performed by addition of a mercaptoalkanesulphonate, optionally followed by addition of a hydroxylamine; step (d) is performed by addition of HCl.

11. Method according to claims 1-10, wherein the oxidative folding is performed by adding the peptide to be folded in a folding buffer, followed by stirring for 1 day at room temperature.

12. Method according to claim 11, wherein the folding buffer is a Tris buffer added with trehalose and guanidine hydrochloride, having final pH in the range 7-9.

13. Method according to claims 1-12, wherein in step c. the dialyzing is performed in a buffer with pH comprised from 4.6 to 8.0, preferably at pH 7.4 ± 0.5.

14. Method according to claim 13, where the buffer is a phosphate buffer.

15. Method according to claims 1-14, wherein the step c. includes a further dialysis in water.

16. Method according to claims 1-15, wherein the final product resulting from step c. is purified by HPLC.

17. Method according to claims 1-16, wherein the target sequence is SEQ.ID.NO: 1 (scFV D2B), SEQ.ID.NO: 2 (scFV Trastuzumab) or SEQ.ID.NO: 3 (scFV Adalimumab).
